# EUROPEAN PATENT SPECIFICATION

(11) **EP 3 466 404 B1**
(45) Date of publication and mention of the grant of the patent: **19.01.2022**
(21) Application number: 17806226.1
(22) Date of filing: 21.04.2017
(51) Int. Cl.: A61K 8/81, A61Q 5/00, A61K 8/04

(54) **HAIR COSMETIC**
HAARKOSMETIKUM
PRODUIT COSMÉTIQUE CAPILLAIRE

(30) Priority: 31.05.2016 JP 2016108131; 31.05.2016 JP 2016108132
(43) Date of publication of application: 10.04.2019
(73) Proprietor: Kao Corporation, Chuo-ku Tokyo 103-8210 (JP)
(72) Inventor: SAITO, Hiroaki, Tokyo 1038210 (JP)
(74) Representative: Hoffmann Eitle
(86) International application number: PCT/JP2017/015978
(87) International publication number: WO 2017/208666

(56) References cited:
- EP-A2- 0 274 086
- WO-A1-00/06092
- WO-A1-99/55952
- JP-A- 2014 097 948
- US-A- 5 686 062
- US-A1- 2007 081 964
- Anonymous: "Luviset CAN Technical Information", , 1 September 2005 (2005-09-01), XP055621903, Retrieved from the Internet: URL:http://www.rumapel.com.ar/cosmetica_po limeros/ficha_tecnica/Luviset%20CAN.pdf [retrieved on 2019-09-13]
- DATABASE GNPD [Online] MINTEL; 20 January 2005 (2005-01-20), anonymous: "Hairspray with Natural Movement", XP55622110, retrieved from www.gnpd.com Database accession no. 10204239
- DATABASE GNPD [Online] MINTEL; 28 February 2013 (2013-02-28), anonymous: "Unscented All Weather Professional Hairspray", XP55622049, retrieved from www.gnpd.com Database accession no. 2009639

## Description

### Field of the Invention

The present invention relates to a hair cosmetic to be used by spraying.

### Background of the Invention

In recent hair-dressing products, the hairstyle is commonly held by the adhesive force of a polymer or oil, or by the strength of a polymer film, and a variety of products are on the market.

Among these, examples of products which fix the hairstyle with a polymer film include atomized cosmetics such as aerosol-type hair spray. An aerosol-type hair spray is usually obtained by dissolving a film-forming resin in an organic solvent such as ethanol and enclosing it in an aerosol can together with a propellant gas. It is generally sprayed on styled hair to fix the hairstyle.

However, in recent years, due to an increased environmental awareness, atomized hair-dressing products not using organic solvents such as alcohol but using water as a solvent are being investigated. For example, Patent Literature 1 describes a hair-setting agent consisting of specific film-forming polymer particles with an average particle diameter of 10-300 nm, containing a carboxyl group neutralized at a neutralization degree of 10-80%, and an aqueous dispersion of a plasticizer. Patent Literature 1 discloses that the hair-dressing product has a low viscosity, dries quickly and has a high fixing force, since the film-forming polymer is not dissolved in the aqueous phase.

On the other hand, various efforts are made to improve the humidity resistance after setting of aqueous hair-dressing products. For example, Patent Literature 2 discloses a gel hairstyling composition containing a first copolymer having a specific structure and a second copolymer having another structure in an aqueous carrier. Patent Literature 2 states that the above hairstyling composition increases the resistance of the hairstyle to humidity, to moisture and to sweat.

(Patent Literature 1) JP-A-7-502761
(Patent Literature 2) JP-A-2008-540461 WO00/06092 teaches compositions for treating hair which are gels, foams or sprays.

### Summary of the Invention

The present invention provides a hair cosmetic to be used by spraying, as defined in claim 1.

### Detailed Description of the Invention

When water is used as a solvent, a set polymer, which is resistant to humidity when used as a hair spray, is difficult to be stably dissolved in water due to its low water solubility. Also, when a water-soluble set polymer is used, the humidity resistance becomes insufficient. Moreover, using a water-soluble set polymer causes the problem that the viscosity increases to prevent a stable discharge in a mist, when the set polymer is incorporated in amounts enough to obtain a sufficient fixing force.

To address this problem, the aqueous cosmetic of Patent Literature 1 utilizes a plasticized pseudolatex in which a polymer having a neutralization ratio reduced to the extent that it is not dissolved in water is dispersed in water, in order to limit the increase in viscosity, shorten the drying time, and increase the fixing force. However, when producing the aqueous cosmetic, the plasticized pseudolatex is prepared by preparing an emulsion adding, while stirring, a water phase in an oil phase in which a film-forming polymer is dissolved homogenously in a volatile organic solvent together with a neutralizer and a plasticizer, then evaporating the total amount of the organic solvent, and is then mixed with the other components, thus requiring a large quantity of a volatile organic solvent and putting a heavy load on the environment.

On the other hand, while being resistant to humidity, the hairstyling composition described in Patent Literature 2 has a problem relating the discharging performance to be used as an aqueous hairspray, since its viscosity increases due to the film-forming resin having an associating group.

Thus, the present invention relates to an atomized hair cosmetic with an excellent durability of the humidity resistance, despite being aqueous, capable of firmly holding the hairstyle even when external physical force is applied, and with good atomizing properties.

As a result of intensive studies, the present inventor found that, by using a specific film-forming polymer and setting it at a specific neutralization degree, the present invention has excellent humidity resistance and can hold firmly the hairstyle even if external force is applied, while being aqueous, having a sufficiently low viscosity and good atomizing properties, and thus the present invention was completed.

The hair cosmetic of the present invention can be discharged as a good mist, has an excellent durability of the humidity resistance while being aqueous, has a high durability of shock resistance thanks to a dry film having both sufficient hardness and softness to physical external forces, and can firmly hold the hairstyle.

### [Component (A): Copolymer comprising the constituent units (a) and (b)]

The copolymer of component (A) has a high humidity resistance after drying and is a polymer relatively difficult to dissolve in water, but by adjusting its neutralization degree, the present invention achieved both humidity resistance and solubility in water.

As a C₃₋₅ unsaturated monocarboxylic acid in the constituent unit (a), acrylic acid, methacrylic acid, crotonic acid are preferable, and as a C₃₋₅ unsaturated dicarboxylic acid, maleic acid is preferable.

In (b1) among the constituent units (b), the fatty acid moiety and the aliphatic moiety of the C₁₋₁₂ fatty acid vinyl ester and the C₁₋₁₂ aliphatic vinyl ether may be either saturated or unsaturated, linear or branched. The number of carbon atoms of the fatty acid moiety and the aliphatic moiety is preferably 2 or more, and preferably 11 or less, more preferably 10 or less.

In (b2) among the constituent units (b), as a C₃₋₅ unsaturated monocarboxylic acid, C₃₋₄ unsaturated carboxylic acid is preferable, with acrylic acid and methacrylic acid being more preferable. Moreover, the C₁₋₆ aliphatic moiety in the constituent unit (b2) is optionally substituted with a hydroxy group, and may be either saturated or unsaturated, linear or branched. Moreover, its number of carbon atoms is 1 or more and 6 or less, but from a viewpoint of discharging the hair cosmetic as a good mist, the number of carbon atoms is preferably 1 or more and more preferably 2 or more. Moreover, the number of carbon atoms is preferably 5 or less and more preferably 4 or less.

The copolymer of component (A) is classified into the following components (A-1) and (A-2) depending on whether the constituent unit (b) is (b1) or (b2).
(A-1): a copolymer comprising the constituent units (a) and (b1)
(A-2): a copolymer comprising the constituent units (a) and (b2)

Specific examples of the copolymer of component (A-1) include a (vinyl acetate/crotonates) copolymer (e.g. Luviset CA66 of BASF) and a (vinyl acetate/crotonates/vinyl neodecanoate) copolymer (e.g. Resyn 28-2930 of AkzoNobel, Luviset CAN of BASF), but the (vinyl acetate/crotonates/vinyl neodecanoate) copolymer is preferable.

Moreover, specific examples of the copolymer of component (A-2) include an acrylates copolymer (e.g. TILAMAR Fix A1000 of DSM, Luvimer 100P of BASF), an acrylates/hydroxyalkyl acrylates copolymer (e.g. ACUDYNE 180 of Dow Chemical), an acrylates/alkyl(C1-2) succinates/hydroxyalkyl acrylates copolymer (e.g. ACUDYNE LT-120 of Dow Chemical) and an acrylates/t-butylacrylamide copolymer (e.g. Ultrahold Strong, Ultrahold 8 of BASF), but the acrylates copolymer, the acrylates/alkyl(C1-2) succinates/hydroxyalkyl acrylates copolymer and the acrylates/t-butylacrylamide copolymer are preferred.

The copolymer of component (A) (A-1) and (A-2).

Moreover, from a viewpoint of the dried film to have good durability of shock resistance and also a solid holding force by being hard enough, while having appropriate softness, the mass ratio of component (A-1) to component (A-2), (A-1)/(A-2), is 0.01 or more, more preferably 0.05 or more, further preferably 0.1 or more, and 5 or less, more preferably 3.0 or less, further preferably 2.0 or less, further preferably 1.5 or less, further preferably 1.3 or less.

From a viewpoint of stably formulating in water, the neutralization degree of component (A) is 50% or more, preferably 52% or more, more preferably 54% or more, further preferably 56% or more, and, from a viewpoint of improving the humidity resistance after drying, is 90% or less, preferably 87% or less, more preferably 83% or less, further preferably 80% or less.

The neutralization degree of component (A) is adjusted by adding a neutralizer, as needed. If the neutralization degree of the raw material copolymer used in component (A) does not reach the above range, the neutralization degree can be increased by adding an alkali agent as a neutralizer, and if the neutralization degree of the raw material copolymer exceeds the above range, the neutralization degree can be decreased by adding an acid as a neutralizer. The amount of neutralizer to be added in order for the neutralization degree to be within the above range can be calculated from the acid value of the raw material copolymer. Among neutralizers, examples of alkali agents include 2-amino-2-methyl-1-propanol, 2-amino-2-methyl-1,3-propanediol, potassium hydroxide, sodium hydroxide, triethanolamine, sodium carbonate, arginine and lysine. Examples of acids include inorganic acids such as phosphoric acid and hydrochloric acid, and organic acids such as glycolic acid, lactic acid, mandelic acid, glyceric acid, pyrrolidone carboxylic acid, gluconic acid, tartaric acid, malic acid and citric acid.

The content of component (A) in the hair cosmetic is preferably 1% by mass or more, more preferably 2% by mass or more, further preferably 3% by mass or more from a viewpoint of having a good durability of the humidity resistance after drying the hair cosmetic of the present invention, and preferably 10% by mass or less, more preferably 8% by mass or less, further preferably 6% by mass or less from a viewpoint of stably dissolving in water and having a good appearance.

### [Component (B) : water]

The water of component (B) is the balance of component (A) and the other optional components, and it content in the hair cosmetic of the present invention is 50% by mass or more, preferably 60% by mass or more, more preferably 70% by mass or more, further preferably 80% by mass or more.

### [Component (C): a plasticizer]

In the hair cosmetic of the present invention, a plasticizer may be added as a component (C) in order to enhance the durability of the shock resistance. Examples of such plasticizer include nonionic surfactants liquid at 25°C such as sorbitol polyoxyalkylene glycol ether, glycerine polyoxyalkylene glycol ether, diglycerine polyoxyalkylene glycol ether, methyl glucose polyoxyalkylene glycol ether, polyoxyalkylene sorbitan fatty acid ester, polyoxyalkylene monoalkyl ether and polyoxyalkylene monoalkenyl ether; and polyhydric alcohols liquid at 25°C such as glycerine, 1,3-butanediol and dipropylene glycol.

The content of component (C) contained in the hair cosmetic of the present invention is preferably 0.01% by mass or more, more preferably 0.05% by mass or more, further preferably 0.1% by mass or more from a viewpoint of enhancing the durability of the shock resistance, and preferably 2% by mass or less, more preferably 1.5% by mass or less, further preferably 1% by mass or less from a viewpoint of enhancing the durability of the humidity resistance.

The mass ratio of component (C) to component (A), (C)/(A), is preferably 0.01 or more, more preferably 0.03 or more, further preferably 0.05 or more from a viewpoint of enhancing the durability of the shock resistance, and preferably 1 or less, more preferably 0.8 or less, further preferably 0.5 or less from a viewpoint of enhancing the durability of the humidity resistance.

### [Component (D): a monohydric alcohol having from 1 to 4 carbon atoms]

The hair cosmetic of the present invention is an aqueous composition with the water of component (B) as a solvent, and from a viewpoint of suppressing the drying speed after spraying and allowing the hairstyle to be arranged as desired until it hardens, preferably does not substantially contain the monohydric alcohol having from 1 to 4 carbon atoms of component (D). "Substantially" means that it excludes the cases where it is unintentionally and inevitably contained as a solvent or the like of the other formulated components. The content of component (D) in the hair cosmetic is preferably 10% by mass or less, more preferably 8% by mass or less, further preferably 6% by mass or less. Moreover, from the same effect, the mass ratio of component (D) to component (B), (D)/(B), is preferably 0 or more and 0.1 or less, more preferably 0 or more and 0.08 or less, further preferably 0 or more and 0.06 or less.

### [Component (E): a polymer type surfactant]

The hair cosmetic of the present invention can also contain a solubilizer of component (A) in order to further improve the solubility in water of component (A) and to obtain a composition having a transparent appearance. Examples of compounds to solubilize component (A) include nonionic surfactants other than those corresponding to component (C), cationic surfactants, anionic surfactants, amphoteric surfactants and polymer type surfactants.

Among the above components, the hair cosmetic of the present invention preferably contains a polymer type surfactant as component (E) from a viewpoint of retaining a transparent appearance, and as a polymer type surfactant, an uncrosslinked polymer containing the following constituent units (e1) and (e2) is preferable.
(e1) a constituent unit derived from a C₃₋₄ unsaturated monocarboxylic acid.
(e2) a constituent unit derived from a C₃₋₅ unsaturated monocarboxylic acid C₁₀₋₃₀ aliphatic ester or a C₃₋₅ unsaturated monocarboxylic acid C₁₀₋₃₀ aliphatic amide.

Since the uncrosslinked polymer of component (E) has a function to disperse in water the copolymer of component (A) without increasing the viscosity of the composition, the hair cosmetic containing the uncrosslinked polymer is considered to be able to retain its transparent appearance.

As a C₃₋₄ unsaturated monocarboxylic acid in the constituent unit (e1), acrylic acid and methacrylic acid are preferable.

As a C₃₋₅ unsaturated monocarboxylic acid in the constituent unit (e2), C₃₋₄ unsaturated carboxylic acid is preferable, acrylic acid, methacrylic acid and crotonic acid are more preferable, and acrylic acid and methacrylic acid are further preferable. Moreover, the C₁₀₋₃₀ aliphatic moiety in the constituent unit (e2) may be either saturated or unsaturated, linear or branched. Moreover, the number of carbon atoms is 10 or more and 30 or less, but it is preferably 14 or more from a viewpoint of dispersing the copolymer of component (A) in water, and preferably 22 or less, more preferably 20 or less, and further preferably 18 or less from a viewpoint of enhancing the solubility and dispersibility in the water of component (E) itself or a lower alcohol.

Moreover, from a viewpoint of stably dissolving component (A) in water and retaining a transparent appearance while having a good mist at discharge and the humidity resistance after the hair cosmetic has dried, the mass ratio of the constituent units (e1)/(e2) in component (E) is preferably 50/50 or more, more preferably 55/45 or more, further preferably 60/40 or more. Moreover, the mass ratio (e1)/(e2) can be 100/0 or less, but from a viewpoint of preventing the film formed on the hair from being too hard and brittle, and of giving it excellent shock resistance, it is preferably 90/10 or less, more preferably 80/20 or less.

Moreover, the polymer of component (E) may have constituent units other than (e1) and (e2), but such constituent units are preferably 30% by mass or less , more preferably 20% by mass or less, further preferably 15% by mass or less of the whole of component (E). Examples of the constituent units other than (e1) and (e2) include nonionic constituent units derived from compounds having a vinyl structure such as vinyl pyrrolidone, vinyl caprolactam and vinly acetate, or compounds having a structure of such as N,N-dialkylacrylamide, (meth)acrylic acid hydroxyalkyl and methoxy PEG (meth)acrylate; cationic constituent units derived from compounds such as N,N-dimethylaminopropyl acrylamide and dimethylaminoethyl acrylamide; anionic constituent units derived from dicarboxylic acids such as itaconic acid, maleic acid and fumaric acid, sulfonic acids such as vinyl sulfonic acid and phosphoric acids such as acryloyloxyethyl phosphate; amphoteric constituent units derived from compounds having a structure such as carboxybetaine, sulfobetaine and phosphobetaine.

Moreover, component (E) is preferably a non-associating polymer.

The production method of this polymer is not particularly limited, but can be produced by making the vinyl monomer capable of forming the above constituent units into a polymer by a known polymerization method such as radical polymerization, living polymerization, living radical polymerization, Group-Transfer polymerization and ring-opening polymerization. The structure of this polymer is not particularly limited and may be any of a random polymer, a block polymer or a graft polymer, but from a viewpoint of stably dissolving component (A) in water and retaining a transparent appearance while having a good mist at discharge, a random polymer is preferable.

From a viewpoint of having a sufficient hardness after the hair cosmetic has dried and excellent humidity resistance, the weight-average molecular weight of component (E) is preferably 5,000 or more, more preferably 7,000 or more, further preferably 10,000 or more. Moreover, from a viewpoint of preventing the film formed on the hair from being too hard and brittle, and of giving it excellent shock resistance, it is preferably 1,000,000 or less, more preferably 100,000 or less, further preferably 50,000 or less.

A specfic example of the copolymer of component (E) is the acrylic acid/stearyl acrylate copolymer.

From a viewpoint of stably dissolving component (A) in water while enhancing the easiness to style until the hair cosmetic dries and the durability of the shock resistance of the hairstyle after drying, the content of component (E) in the hair cosmetic of the present invention is preferably 0.01% by mass or more, more preferably 0.05% by mass or more, further preferably 0.10% by mass or more, and from a viewpoint of appropriate softness of the dried film, the content is preferably 1% by mass or less, more preferably 0.8% by mass or less, further preferably 0.6% by mass or less.

From a viewpoint of enhancing the humidity resistance after drying, the mass ratio of component (E) to component (A) in the hair cosmetic of the present invention, (E)/(A), is 0.001 or more, preferably 0.005 or more, more preferably 0.01 or more, further preferably 0.02 or more, and from a viewpoint of stably dissolving in water, the mass ratio is 0.5 or less, preferably 0.4 or less, more preferably 0.3 or less, further preferably 0.2 or less, further preferably 0.1 or less.

### [Other components]

In addition to the above-mentioned components, the hair cosmetic of the present invention can be formulated with components used in normal hair cosmetics depending on the purpose. Examples of such components include set polymers other than component (A), anti-dandruff agents, vitamins, bactericides, anti-inflammatory agents, chelating agents, moisturizers (such as panthenol), pH regulators, colorants such as dyes and pigments, plant extracts, pearling agents, fragrances, ultraviolet absorbing agents and antioxidants. Each of these agents is not limited to its use as an agent, and can be diverted to other uses depending on the purpose, for example, a bactericide can be used as an anti-dandruff agent. Or, it can be used for other uses simultaneously, for example it can have an effect as a bactericide and as an anti-dandruff agent.

### [Dosage form]

As a dosage form of the hair cosmetic of the present invention, any form is possible as long as it is sprayable, for example, aerosol types and non-aerosol types (pump spray, pressure accumulation spray, etc.) are possible, but non-aerosol types are preferable.

### [Viscosity]

From a viewpoint of allowing to spray as a good mist, while also reducing problems such as the clogging of the nozzle of the spraying device, the viscosity of the hair cosmetic of the present invention is preferably 0.1 mPa·s or more and 100 mPa·s or less, more preferably 0.1 mPa·s or more and 20 mPa·s or less, further preferably 0.1 mPa·s or more and 10 mPa·s or less.

The viscosity is the value after rotating for 1 min at 30 rpm with Type B rotational viscometer (model TVB-10H) of Toki Sangyo Co.,Ltd. using the rotor No.19 (L/Adp). The measurement is conducted in a thermostat bath at 25°C.

### [pH]

The pH of the hair cosmetic of the present invention is measured using a glass-electrode hydrogen ion densitometer F-14 (Horiba, Ltd.), by adjusting the sample temperature to 25°C and directly inserting the electrode in the sample. The pH of the hair cosmetic of the present invention is preferably 2 or more, more preferably 3 or more, and preferably 9 or less, more preferably 8 or less.

With regard to the embodiments mentioned above, the preferred aspects of the present disclosure are further elaborated below.
<1> A hair cosmetic to be used by spraying, comprising the following components (A) and (B), with a content of the component (B) being 50% by mass or more:
   (A) a non-associating copolymer comprising constituent units (a) and (b) and having a neutralization degree of 50% or more and 90% or less,
      (a) a constituent unit derived from a C₃₋₅ unsaturated mono- or dicarboxylic acid,
      (b) at least one selected from the following (b1) and (b2):
         (b1) a constituent unit derived from a C₁₋₁₂ fatty acid vinyl ester or a C₁₋₁₂ aliphatic vinyl ether;
         (b2) a constituent unit derived from a C₃₋₅ unsaturated monocarboxylic acid C₁₋₆ aliphatic ester or a C₃₋₅ unsaturated monocarboxylic acid C₁₋₆ aliphatic amide, which is optionally substituted with a hydroxy group;
   (B) water.
<2> The hair cosmetic according to <1>, wherein the component (A) is preferably one or two selected from the following components (A-1) and (A-2), and more preferably comprising both components (A-1) and (A-2):
   (A-1) a copolymer comprising the constituent units (a) and (b-1);
   (A-2) a copolymer comprising the constituent units (a) and (b-2).
<3> The hair cosmetic according to <2>, wherein a mass ratio of component (A-1) to component (A-2), (A-1)/(A-2), is preferably 0.01 or more, more preferably 0.05 or more, further preferably 0.1 or more, and preferably 5 or less, more preferably 3.0 or less, further preferably 2.0 or less, further preferably 1.5 or less, further preferably 1.3 or less.
<4> The hair cosmetic according to any one of <1> to <3>, further comprising the following component (C):
   (C) a plasticizer.
<5> The hair cosmetic according to <4>, wherein a mass ratio of component (C) to component (A), (C)/(A), is preferably 0.01 or more, more preferably 0.03 or more, further preferably 0.05 or more, and preferably 1 or less, more preferably 0.8 or less, further preferably 0.5 or less.
<6> The hair cosmetic according to <4> or <5>, wherein the component (C) is preferably a nonionic surfactant liquid at 25°C and a polyhydric alcohol liquid at 25°C, more preferably one or two or more selected from the group consisting of sorbitol polyoxyalkylene glycol ether, glycerine polyoxyalkylene glycol ether, diglycerine polyoxyalkylene glycol ether, methyl glucose polyoxyalkylene glycol ether, polyoxyalkylene sorbitan fatty acid ester, polyoxyalkylene monoalkyl ether, polyoxyalkylene monoalkenyl ether, glycerine, 1,3-butanediol and dipropylene glycol.
<7> The hair cosmetic according to any one of <1> to <6>, wherein a content of the component (D) is preferably 10 % by mass or less, more preferably 8% by mass or less, further preferably 6% by mass or less:
   (D) a monohydric alcohol having from 1 to 4 carbon atoms.
<8> The hair cosmetic according to <7>, wherein a mass ratio of component (D) to component (B), (D)/(B), is preferably 0 or more and 0.1 or less, more preferably 0 or more and 0.08 or less, further preferably 0 or more and 0.06 or less.
<9> The hair cosmetic according to any one of <1> to <8>, wherein a neutralization degree of component (A) is preferably 52% or more, more preferably 54% or more, further preferably 56% or more, and preferably 87% or less, more preferably 83% or less, further preferably 80% or less.
<10> The hair cosmetic according to any one of <2> to <9>, wherein the component (A-1) is preferably a (vinyl acetate/crotonates) copolymer or a (vinyl acetate /crotonates/vinyl neodecanoate) copolymer, more preferably a (vinyl acetate/crotonates/vinyl neodecanoate) copolymer.
<11> The hair cosmetic according to any one of <2> to <10>, wherein the component (A-2) is preferably an acrylates copolymer, an (acrylates/hydroxyalkyl acrylates) copolymer, an (acrylates/alkyl(C1-2) succinates/hydroxyalkyl acrylates) copolymer or an (acrylates/t-butylacrylamide) copolymer, more preferably an acrylates copolymer, an (acrylates/alkyl(C1-2) succinates/hydroxyalkyl acrylates) copolymer or an (acrylates/t-butylacrylamide) copolymer.
<12> The hair cosmetic according to any one of <1> to <11>, wherein the component (A) is preferably dissolved.
<13> The hair cosmetic according to any one of <1> to <12>, preferably further comprising a polymer type surfactant as component (E).
<14> The hair cosmetic according to <13>, wherein the polymer type surfactant of the component (E) is an uncrosslinked polymer comprising the following constituent units (e1) and (e2):
   (e1) a constituent unit derived from a C₃₋₄ unsaturated monocarboxylic acid.
   (e2) a constituent unit derived from a C₃₋₅ unsaturated monocarboxylic acid C₁₀₋₃₀ aliphatic ester or a C₃₋₅ unsaturated monocarboxylic acid C₁₀₋₃₀ aliphatic amide.
<15> The hair cosmetic according to <14>, wherein a mass ratio of constituent units (e1)/(e2) in component (E) is preferably 50/50 or more, more preferably 55/45 or more, further preferably 60/40 or more, and 100/0 or less, preferably 90/10 or less, more preferably 80/20 or less.
<16> The hair cosmetic according to <14> or <15>, wherein constituent units other than (e1) and (e2) comprised in component (E) are preferably 30% by mass or less, more preferably 20% by mass or less, further preferably 15% by mass or less of the whole of component (E).
<17> The hair cosmetic according to any one of <13> to <16>, wherein a mass ratio of component (E) to component (A), (E)/(A), is preferably 0.005 or more, more preferably 0.01 or more, further preferably 0.02 or more, and preferably 0.4 or less, more preferably 0.3 or less, further preferably 0.2 or less, further preferably 0.1 or less.
<18> The hair cosmetic according to any one of <13> to <17>, wherein a content of the component (E) is preferably 0.01% by mass or more, more preferably 0.05% by mass or more, further preferably 0.10% by mass or more, and preferably 1% by mass or less, more preferably 0.8% by mass or less, further preferably 0.6% by mass or less.
<19> The hair cosmetic according to any one of <13> to <18>, wherein a weight-average molecular weight of component (E) is preferably 5,000 or more, more preferably 7,000 or more, further preferably 10,000 or more, and preferably 1,000,000 or less, more preferably 100,000 or less, further preferably 50,000 or less.
<20> The hair cosmetic according to any one of <1> to <19>, preferably being a non-aerosol type.
<21> A hair cosmetic to be used by spraying, comprising the following components (A), (B), (C) and (E), with a content of the component (B) being 50% by mass or more, a mass ratio of component (C) to component (A), (C)/(A), being 0.03 or more and 0.8 or less and a mass ratio of component (E) to component (A), (E)/(A), being 0.01 or more and 0.2 or less:
   (A) a combination of components (A-1) and (A-2),
   wherein a mass ratio (A-1)/(A-2) is 0.05 or more and 3.0 or less:
   Component (A-1): a non-associating copolymer comprising constituent units (a) and (b1) and having a neutralization degree of 50 or more and 90% or less,
   Component (A-2): a non-associating copolymer comprising constituent units (a) and (b2) and having a neutralization degree of 50 or more and 90% or less:
      (a) a constituent unit derived from a C₃₋₅ unsaturated mono- or dicarboxylic acid,
      (b1) a constituent unit derived from a C₁₋₁₂ fatty acid vinyl ester or a C₁₋₁₂ aliphatic vinyl ether,
      (b2) a constituent unit derived from a C₃₋₅ unsaturated monocarboxylic acid C₁₋₆ aliphatic ester or a C₃₋₅ unsaturated monocarboxylic acid C₁₋₆ aliphatic amide, which is optionally substituted with a hydroxy group;
         (B) water;
         (C) a plasticizer;
         (E) a polymer type surfactant that is an uncrosslinked polymer comprising the following constituent units (e1) and (e2):
            (e1) a constituent unit derived from a C₃₋₄ unsaturated monocarboxylic acid;
            (e2) a constituent unit derived from a C₃₋₅ unsaturated monocarboxylic acid C₁₀₋₃₀ aliphatic ester or a C₃₋₅ unsaturated monocarboxylic acid C₁₀₋₃₀ aliphatic amide.

### Examples

### Examples 1-15, Comparative Examples 1-2

The aqueous hair cosmetics shown in Table 1 and Table 2 were prepared and various evaluations were conducted according to the following methods. The results are also shown in Table 1 and Table 2.

In the Comparative Example 1, as described in the table, the discharge as a mist by a pump was not possible due to insoluble matter and thus nothing was evaluated except the appearance.

### [Evaluation methods]

### <Appearance of composition>

20 g of each composition was put into a transparent screw tube No.6 from Maruemu, a paper with black and white stripes at 4 mm intervals was placed behind the screw tube and it was determined according to the following criteria whether the striped pattern in the background was visible or not when visually observing each composition from the front.

White turbidity: the composition is white and cloudy, and the striped pattern is invisible

Slight turbidity: the composition is white and cloudy, but the striped pattern is visible

Transparent: the composition is not cloudy and the striped pattern is visible

### <State of mist>

A non-aerosol type spray was prepared by putting 20 g of each composition into a transparent screw tube No.6 from Maruemu and attaching a pump YSR-0.3L from Yoshino Seisakusho. Each non-aerosol type spray was pushed 5 times, and the state of the mist was visually observed and evaluated on a three-point scale "good mist" / "coarse mist" / "does not become a mist".

### <Hardness of tress>

After spraying 0.5 g of each non-aerosol type spray on Chinese non-chemically treated straight hair tresses of a length of 25 cm and a weight of 3 g, the hair was styled by hand combing in a straight line shape and dried for 1 hour under the laboratory atmosphere. A sensory evaluation was conducted on the hardness of the tress after drying. The evaluation was carried out by 7 panelists who alternatively selected either "hard" / "cannot say" / "soft". The number of panelists who answered "hard" / "cannot say" / "soft" is shown in this order.

### <Durability of humidity resistance>

Chinese non-chemically treated straight hair tresses of a length of 25 cm and a weight of 3 g were wetted with water, and after rolling them on a resin-made rod of a diameter of 2.2 cm and a length of 9 cm and drying them for 1 hour in an oven at 60°C, they were allowed to stand for 1 hour under the laboratory atmosphere (25°C, 50% RH. All the same hereinafter.). After removing the tresses from the rod, the tresses set in a waved shape were prepared by slightly detangling them by passing Nakajima Sangyo's set comb (L) AS-600 once from the roots to the ends of the tresses.

1.0 g of each non-aerosol type spraying agent used for the evaluation of the mist's state was sprayed on the above wave-shaped tresses and was air-dried for 1 hour under the laboratory atmosphere. Then, they were allowed to stand for 30 minutes in a high humidity environment of 90% RH at 25°C and the durability of the humidity resistance was determined from the length of the tress after 30 minutes according to the following criteria.
Score 5: less than 19.5 cm
Score 4: 19.5 to less than 21 cm
Score 3: 21 to less than 22.5 cm
Score 2: 22.5 to less than 24 cm
Score 1: 24 cm or more

### <Durability of shock resistance>

1.0 g of each non-aerosol type spray was sprayed on the tresses set in a waved shape prepared according to the same method as the above evaluation of the humidity resistant holding property, and was air-dried for 1 hour under the laboratory atmosphere.

Then, the state of the tresses after gripping them by the hand from below 10 times in a row was observed and evaluated whether the shape was maintained without any change such as falling apart. The evaluation was conducted by 7 panelists who alternatively selected either "the shape is maintained" / "cannot say" / "the shape is lost". The number of panelists who answered "the shape is maintained" / "cannot say" / "the shape is lost" is shown in order.

### <Evaluation method of the easiness to style>

The evaluation was conducted by 7 women panelists with long hair. The curly style was made by rolling the tresses on a curling iron of a diameter of 32 mm and a surface temperature of 180°C (Create Ion J72010M), and after holding for 10 seconds, by repeatedly removing the iron from the tresses on the whole hair. Next, after spraying 3.0 g of each non-aerosol type spray, the easiness to style was evaluated by lightly styling the hair with the hands before the sprayed composition dried. The evaluation was conducted by 7 panelists, who alternatively selected either "easy to style" / "cannot say" / "difficult to style". The number of panelists who answered "easy to style" / "cannot say" / "difficult to style" is shown in this order.

### <Evaluation method of the maintenance of the transparent appearance>

20 g of each composition was put into a Maruemu transparent screw tube No.6 and the appearance of the composition after leaving it for a month at room temperature was determined according to the following criteria.
A: transparent without change from the beginning
B: the appearance has changed, e.g. presence of a deposit, and the transparency is lost (including when it is not transparent from the beginning)

**[Table 1]**

| (% by mass; active amount) | | Examples | | | | | | Comparative Examples | |
|---|---|---|---|---|---|---|---|---|---|
| | | 1 | 2 | 3 | 4 | 5 | 6 | 1 | 2 |
| (A-1) | (VA/crotonates/vinyl neodecanoate) copolymer (*1) | 0.6 | 0.6 | 1.6 | 1.6 | 2 | 2 | 0.6 | 0.6 |
| (A-2) | Acrylates copolymer (*2) | 3.3 | 3.3 | 1.52 | 1.52 | 1.9 | 1.9 | 3.3 | 3.3 |
| | (Acrylates/t-butylacrylamide) copolymer (*3) | | | | | | | - | - |
| | (Acrylates/alkyl(C1-2) succinates/hydroxyalkyl acrylates) copolymer (*4) | - | - | - | - | - | - | - | - |
| (E) | (Acrylic acid/stearyl acrylate) copolymer (*5) | 0.1 | 0.1 | 0.08 | 0.08 | 0.1 | 0.1 | 0.1 | 0.1 |
| | 2-amino-2-methyl-1-propanol | 0.63 | 0.63 | 0.41 | 0.41 | 0.52 | 0.52 | 0.32 | 0.76 |
| (B) | Water | bal. | bal. | bal. | bal. | bal. | bal. | bal. | bal. |
| (C) | (C12-14) Sec-Pareth-9 (*6) | 0.3 | 0.3 | 0.6 | 0.6 | 0.5 | 0.5 | 0.3 | 0.3 |
| | Polysorbate 20 (*8) | - | - | - | - | - | - | - | - |
| | Dipropylene glycol | 0.1 | - | 0.1 | - | 0.1 | - | 0.1 | 0.1 |
| | PEG-40 Hydrogenated Castor oil (*7) | 0.4 | 0.4 | 0.32 | 0.32 | 0.4 | 0.4 | 0.4 | 0.4 |
| Mass ratio | (A-1)/(A-2) | 0.18 | 0.18 | 1.05 | 1.05 | 1.05 | 1.05 | 0.18 | 0.18 |
| | (C)/(A) | 0.10 | 0.08 | 0.22 | 0.19 | 0.15 | 0.13 | 0.10 | 0.10 |
| | (E)/(A) | 0.026 | 0.026 | 0.026 | 0.026 | 0.026 | 0.026 | 0.026 | 0.026 |
| Neutralization degree | | 75 | 75 | 75 | 75 | 75 | 75 | 40 | 100 |
| pH | | 7.9 | 7.9 | 7.8 | 7.8 | 7.7 | 7.7 | Not measured | 8.9 |
| Viscosity (mPa·s) | | 1.6 | 1.6 | 1.5 | 1.5 | 1.6 | 1.6 | | 2 |
| Evaluation | Appearance of the formulated liquid | Transparent | Transparent | Transparent | Transparent | Transparent | Transparent | Insoluble | Transparent |
| | Tress hardness | 7/0/0 | 7/0/0 | 5/2/0 | 5/2/0 | 6/1/0 | 6/1/0 | Not evaluated | 6/1/0 |
| | Durability of the humidity resistance | 5 | 5 | 4 | 4 | 5 | 5 | | 2 |
| | Durability of the shock resistance the shock resistance | 6/1/0 | 6/1/0 | 5/2/0 | 5/2/0 | 7/0/0 | 7/0/0 | | 6/1/0 |
| | State of the mist | Good | Good | Good | Good | Good | Good | | Good |
| | Easiness to style | 7/0/0 | 7/0/0 | 5/2/0 | 5/2/0 | 7/0/0 | 7/0/0 | | 7/0/0 |
| | Maintenance of the transparent appearance | A | A | A | A | A | A | | A |

**[Table 2]**

| (% by mass; active amount) | | Examples | | | | | | | | |
|---|---|---|---|---|---|---|---|---|---|---|
| | | 7 | 8 | 9 | 10 | 11 | 12 | 13 | 14 | 15 |
| (A-1) | (VA/crotonates/vinyl neodecanoate) copolymer (*1) | 2 | 2 | 2.2 | 2.2 | 2.2 | 2.2 | 2.2 | 2.2 | 2.2 |
| (A-2) | Acrylates copolymer (*2) | - | - | - | - | - | - | - | - | - |
| | (Acrylates/t-butylacrylamide) copolymer (*3) | - | - | 1.8 | 1.7 | 1.7 | 1.6 | 1.5 | 1.5 | 1.4 |
| | (Acrylates/alkyl(C1-2) succinates/hydroxyalkyl acrylates) copolymer (*4) | 1.6 | 1.6 | - | - | - | - | - | - | - |
| (E) | (Acrylic acid/stearyl acrylate) copolymer (*5) | 0.4 | 0.4 | - | 0.1 | 0.1 | 0.2 | 0.3 | 0.3 | 0.4 |
| | 2-amino-2-methyl-1-propanol | 0.47 | 0.47 | 0.4 | 0.4 | 0.4 | 0.4 | 0.5 | 0.5 | 0.56 |
| (B) | Water | bal. | bal. | bal. | bal. | bal. | bal. | bal. | bal. | bal. |
| (C) | C12-14 Sec-Pareth-9 (*6) | 0.15 | 0.15 | 0.15 | 0.15 | 0.15 | 0.15 | 0.15 | 0.15 | 0.15 |
| | Polysorbate 20 (*8) | 0.1 | 0.1 | - | - | - | - | - | - | - |
| | Dipropylene glycol | 0.1 | - | 0.1 | 0.1 | - | - | 0.1 | - | 0.1 |
| | PEG-40 Hydroqenated Castor oil (*7) | 0.4 | 0.4 | 0.4 | 0.4 | 0.4 | 0.4 | 0.4 | 0.4 | 0.4 |
| Mass ratio | (A-1)/(A-2) | 1.25 | 1.25 | 1.22 | 1.29 | 1.29 | 1.38 | 1.47 | 1.47 | 1.57 |
| | (C)/(A) | 0.10 | 0.07 | 0.06 | 0.06 | 0.04 | 0.04 | 0.07 | 0.04 | 0.07 |
| | (E)/(A) | 0.11 | 0.111 | 0 | 0.026 | 0.026 | 0.053 | 0.081 | 0.081 | 0.11 |
| Neutralization degree | | 56 | 56 | 80 | 80 | 80 | 80 | 80 | 80 | 80 |
| pH | | 6.9 | 6.9 | 7.2 | 7.1 | 7.1 | 7.1 | 7.1 | 7.1 | 7.2 |
| Viscosity (mPa·s) | | 1.7 | 1.7 | 1.9 | 1.9 | 1.9 | 1.9 | 1.9 | 1.9 | 1.9 |
| Evaluation | Appearance of the formulated liquid | Transparent | Transparent | Cloudy | Transparent | Transparent | Transparent | Transparent | Transparent | Transparent |
| | Tress hardness | 6/1/0 | 6/1/0 | 5/2/0 | 6/1/0 | 6/1/0 | 5/2/0 | 5/2/0 | 5/2/0 | 5/2/0 |
| | Durability of the humidity resistance | 5 | 5 | 4 | 4 | 4 | 4 | 4 | 4 | 4 |
| | Durability of the shock resistance | 7/0/0 | 7/0/0 | 6/1/0 | 6/1/0 | 6/1/0 | 6/1/0 | 6/1/0 | 6/1/0 | 5/2/0 |
| | State of the mist | Good | Good | Good | Good | Good | Good | Good | Good | Good |
| | Easiness to style | 7/0/0 | 7/0/0 | 7/0/0 | 7/0/0 | 7/0/0 | 7/0/0 | 7/0/0 | 7/0/0 | 7/0/0 |
| | Maintenance of the transparent appearance | A | A | B | A | A | A | A | A | A |

*1: Resyn 28-2930 (AkzoNobel)
*2: TILAMAR Fix A1000 (DSM)
*3: Ultrahold Strong (BASF)
*4: ACUDYNE LT-120 (Dow Chemical)
*5: Polymer with a molecular weight of 20,000, wherein the acrylic acid:stearyl acrylate ratio is 67:33
*6: SOFTANOL 90 (Nippon Shokubai)
*7: EMANON CH-40 (Kao)
*8: RHEODOL TW-L120 (Kao)

## Claims

1. A hair cosmetic to be used by spraying, comprising the following components (A) and (B), with a content of the component (B) being 50% by mass or more:
(A) a non-associating copolymer comprising constituent units (a) and (b) and having a neutralization degree of 50% or more and 90% or less:
(a) a constituent unit derived from a C₃₋₅ unsaturated mono- or dicarboxylic acid,
(b) at least one selected from the following (b1) and (b2):
(b1) a constituent unit derived from a C₁₋₁₂ fatty acid vinyl ester or a C₁₋₁₂ aliphatic vinyl ether,
(b2) a constituent unit derived from a C₃₋₅ unsaturated monocarboxylic acid C₁₋₆ aliphatic ester or a C₃₋₅ unsaturated monocarboxylic acid C₁₋₆ aliphatic amide, which is optionally substituted with a hydroxy group,
wherein component (A) comprises both of the following components (A-1) and (A-2) in combination:
(A-1) a copolymer comprising the constituent units (a) and (b-1),
(A-2) a copolymer comprising the constituent units (a) and (b-2) and wherein a mass ratio of the component (A-1) to the component (A-2), (A-1)/(A-2), is 0.01 or more and 5 or less;
(B) water.

2. The hair cosmetic according to claim 1, further comprising the following component (C):
(C) a plasticizer.

3. The hair cosmetic according to claim 2, wherein a mass ratio of the component (C) to the component (A), (C)/(A), is 0.01 or more and 1 or less.

4. The hair cosmetic according to claim 2 or 3, wherein the component (C) is selected from nonionic surfactants liquid at 25°C and polyhydric alcohols liquid at 25°C.

5. The hair cosmetic according to any one of claims 1 to 4, wherein a content of the following component (D) is 10 % by mass or less:
(D) a monohydric alcohol having 1 to 4 carbon atoms.

6. The hair cosmetic according to claim 5, wherein a mass ratio of the component (D) to the component (B), (D)/(B), is 0 or more and 0.1 or less.

7. The hair cosmetic according to any one of claims 1 to 6, having a transparent appearance.

8. The hair cosmetic according to any one of claims 1 to 7, being a non-aerosol type.

## Patentansprüche

1. Haarkosmetikum zur Verwendung durch Sprühen, umfassend die folgenden Komponenten (A) und (B), wobei der Gehalt der Komponente (B) 50 Massen-% oder mehr beträgt:
(A) ein nicht-assoziierendes Copolymer, welches die Grundeinheiten (a) und (b) umfasst und einen Neutralisationsgrad von 50 % oder mehr und 90 % oder weniger aufweist:
(a) eine von einer ungesättigten C₃₋₅-Mono- oder Dicarbonsäure abgeleitete Grundeinheit,
(b) mindestens eines, ausgewählt aus den folgenden (b1) und (b2):
(b1) eine von einem C₁₋₁₂-Fettsäurevinylester oder einem aliphatischen C₁₋₁₂-Vinylether abgeleiteten Grundeinheit,
(b2) eine von einem ungesättigten C₃₋₅-Monocarbonsäure-C₁₋₆-aliphatischen Ester oder einem ungesättigten C₃₋₅-Monocarbonsäure-C₁₋₆-aliphatischen Amid ableiteten Grundeinheit, die optional mit einer Hydroxygruppe substituiert ist,
wobei die Komponente (A) beide der folgenden Komponenten (A-1) und (A-2) in Kombination umfasst:
(A-1) ein Copolymer, umfassend die Grundeinheiten (a) und (b-1),
(A-2) ein Copolymer, umfassend die Grundeinheiten (a) und (b-2), und wobei das Massenverhältnis der Komponente (A-1) zur Komponente (A-2), (A-1)/(A-2), 0,01 oder mehr und 5 oder weniger beträgt;
(B) Wasser.

2. Haarkosmetikum gemäß Anspruch 1, ferner umfassend die folgende Komponente (C):
(C) einen Weichmacher.

3. Haarkosmetikum gemäß Anspruch 2, wobei ein Massenverhältnis der Komponente (C) zur Komponente (A), (C)/(A), 0,01 oder mehr und 1 oder weniger beträgt.

4. Haarkosmetikum gemäß Anspruch 2 oder 3, wobei die Komponente (C) ausgewählt ist aus bei 25°C flüssigen nichtionischen Tensiden und bei 25°C flüssigen mehrwertigen Alkoholen.

5. Haarkosmetikum gemäß mindestens einem der Ansprüche 1 bis 4, wobei ein Gehalt der folgenden Komponente (D) 10 Massen-% oder weniger beträgt:
(D) ein einwertiger Alkohol mit 1 bis 4 Kohlenstoffatomen.

6. Haarkosmetikum gemäß Anspruch 5, wobei ein Massenverhältnis der Komponente (D) zur Komponente (B), (D)/(B), 0 oder mehr und 0,1 oder weniger beträgt.

7. Haarkosmetikum gemäß mindestens einem der Ansprüche 1 bis 6, wobei dieses ein transparentes Aussehen aufweist.

8. Haarkosmetikum gemäß mindestens einem der Ansprüche 1 bis 7, wobei dieses von einem aerosolfreien Typ ist.

## Revendications

1. Produit cosmétique capillaire à utiliser par pulvérisation, comprenant les composants (A) et (B) suivants, une teneur en composant (B) étant supérieure ou égale à 50 % en masse :
(A) un copolymère non associatif comprenant des motifs constitutifs (a) et (b) et présentant un degré de neutralisation supérieur ou égal à 50 % et inférieur ou égal à 90%:
(a) un motif constitutif issu d'un acide mono- ou dicarboxylique insaturé en C3-5,
(b) au moins l'un sélectionné parmi les (b1) et (b2) suivants :
(b1) un motif constitutif issu d'un ester vinylique d'acide gras en C₁₋₁₂ ou d'un éther vinylique aliphatique en C₁₋₁₂,
(b2) un motif constitutif issu d'un ester aliphatique en C₁₋₆ d'un acide monocarboxylique insaturé en C₃₋₅ ou d'un amide aliphatique en C₁₋₆ d'un acide monocarboxylique insaturé en C₃₋₅, qui est éventuellement substitué par un groupe hydroxy,
dans lequel le composant (A) comprend à la fois les composants (A-1) et (A-2) suivants en combinaison :
(A-1) un copolymère comprenant les motifs constitutifs (a) et (b-1),
(A-2) un copolymère comprenant les motifs constitutifs (a) et (b- 2) et dans lequel un rapport massique du composant (A-1) au composant (A-2), (A-1)/(A-2), est supérieur ou égal à 0,01 et inférieur ou égal à 5 ;
(B) de l'eau.

2. Produit cosmétique capillaire selon la revendication 1, comprenant en outre le composant (C) suivant :
(C) un plastifiant.

3. Produit cosmétique capillaire selon la revendication 2, dans lequel un rapport massique du composant (C) au composant (A), (C)/(A), est supérieur ou égal à 0,01 et inférieur ou égal à 1.

4. Produit cosmétique capillaire selon la revendication 2 ou 3, dans lequel le composant (C) est sélectionné parmi des tensioactifs non ioniques liquides à 25 °C et des polyols liquides à 25 °C.

5. Produit cosmétique capillaire selon l'une quelconque des revendications 1 à 4, dans lequel une teneur en composant (D) suivant est inférieure ou égale à 10 % en masse :
(D) un alcool monohydrique présentant de 1 à 4 atomes de carbone.

6. Produit cosmétique capillaire selon la revendication 5, dans lequel un rapport massique du composant (D) au composant (B), (D)/(B), est supérieur ou égal à 0 et inférieur ou égal à 0,1.

7. Produit cosmétique capillaire selon l'une quelconque des revendications 1 à 6, présentant un aspect transparent.

8. Produit cosmétique capillaire selon l'une quelconque des revendications 1 à 7, étant d'un type non aérosol.
